(19) Europäisches Patentamt
European Patent Office
Office européen des brevets

(11) Veröffentlichungsnummer: **0 046 496**
**B1**

# (12) EUROPÄISCHE PATENTSCHRIFT

(45) Veröffentlichungstag der Patentschrift: **31.08.83**

(51) Int. Cl.[3]: **C 07 C 69/16**, C 07 C 67/05

(21) Anmeldenummer: **81105377.6**

(22) Anmeldetag: **10.07.81**

(54) **Verfahren zur Herstellung von Diacyloxyhexadienen aus Hexatrienen und neue Diacyloxyhexadiene.**

(30) Priorität: **16.08.80 DE 3030997**

(43) Veröffentlichungstag der Anmeldung: **03.03.82 Patentblatt 82/9**

(45) Bekanntmachung des Hinweises auf die Patenterteilung: **31.08.83 Patentblatt 83/35**

(84) Benannte Vertragsstaaten: **CH DE FR GB IT LI**

(56) Entgegenhaltungen: **DE-A-2 415 248**

(73) Patentinhaber: **BASF Aktiengesellschaft, Carl-Bosch-Strasse 38, D-6700 Ludwigshafen (DE)**

(72) Erfinder: **Weitz, Hans-Martin, Dr., Auf dem Koeppel 40, D-6702 Bad Duerkheim (DE)**
Erfinder: **Fischer, Rolf, Dr., Bergstrasse 98, D-6900 Heidelberg (DE)**

**EP 0 046 496 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Diacyloxyhexadienen aus Hexatrienen und neue Diacyloxyhexadiene

Diese Erfindung betrifft ein Verfahren zur Herstellung von Diacyloxyhexadienen durch Umsetzung von Hexatrienen mit Sauerstoff und Carbonsäuren in Gegenwart von Katalysatoren.

Es ist bekannt, dass sich 1.3-Butadien, das durch Alkyl- oder Acyloxygruppen substituiert sein kann, mit Sauerstoff und Carbonsäuren in Gegenwart von Palladium oder Platin enthaltenden Katalysatoren zu 2-Buten-1.4-diol-diestern und 1-Buten-3.4-diol-diestern umsetzen lässt. Hierfür können homogen gelöste Katalysatoren oder Trägerkatalysatoren verwendet werden.

So erhält man beispielsweise nach Angaben der GB-PS 1 138 366 durch Umsetzung von Piperylen mit Luft in Gegenwart von in Eisessig gelöstem Palladiumacetat, Kupferacetat und Lithiumacetat Gemische aus 1.4-Diacetoxy-2-penten und 4.5-Diacetoxy-2-penten im Molverhältnis 4:1. In ähnlicher Weise wird 1.3-Butadien nach der GB-PS 1 277 837 mit Sauerstoff in Gegenwart von in Eisessig gelöstem Palladiumacetat, Kupferacetat und Kaliumacetat zu 1.4-Diacetoxy-2-buten umgesetzt.

In der DE-PS 2 217 452 wird ein Verfahren zur Acetoxilierung von Butadien beschrieben, bei dem man Palladium-Trägerkatalysatoren verwendet, die zusätzlich Antimon, Bismut, Tellur oder Selen enthalten. Die in der DE-OS 2 728 574 beschriebene Acetoxilierung führt man in Gegenwart von schwefelhaltigen Palladium-Trägerkatalysatoren durch, die ausserdem noch andere Elemente enthalten können. Nach der DE-OS 2 820 519 verwendet man Trägerkatalysatoren, die Palladium und Kupfer in Form einer intermetallischen Verbindung enthalten. Die DE-OS 2 417 558 beschreibt Platin-Trägerkatalysatoren, die zusätzlich eines der Elemente Phosphor, Arsen, Antimon, Selen oder Tellur enthalten. Bei der Umsetzung von Butadien mit Sauerstoff und Essigsäure («Acetoxilierung») entstehen in Gegenwart der genannten Katalysatoren ganz überwiegend cis- und trans-2-Buten-1.4-dioldiacetate. 1-Buten-3.4-diolmono- und 1-Buten-3.4-dioldiacetate werden nur in untergeordnetem Masse gebildet.

Es wurde nun überraschenderweise gefunden, dass man bei der Umsetzung von 1.3.5-Hexatrienen in Gegenwart von Palladium, Platin oder Salzen dieser Metalle mit Sauerstoff und Carbonsäuren 1.6-Diacyloxy-2.4-hexadiene erhält.

Nach dem erfindungsgemässen Verfahren werden Diacyloxyhexadiene der Formel

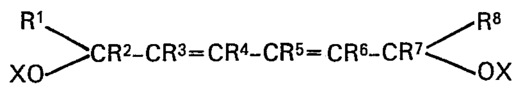 I,

in der X einen $R^9$-CO-Rest und die Reste $R^1$ bis $R^9$ Wasserstoffatome oder Alkylreste mit 1 bis 3 C-Atomen bedeuten, dadurch erhalten, dass man Hexatriene der Formel

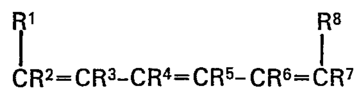 II

in Gegenwart von Katalysatoren, die Palladium, Platin oder Salze dieser Metalle enthalten, mit Sauerstoff und Carbonsäuren der Formel $R^9COOH$ umsetzt.

Die Reaktion kann für den Fall der Umsetzung von 2.4.6-Octatrien (1.6-Dimethyl-1.3.5-hexatrien) mit Essigsäure und Sauerstoff zu 2.7-Diacetoxy-3.5-octadien (1.6-Dimethyl-1.6-diacetoxy-2.4-hexadien) durch die folgenden Formeln wiedergegeben werden (-OAc= -O-CO-$CH_3$):

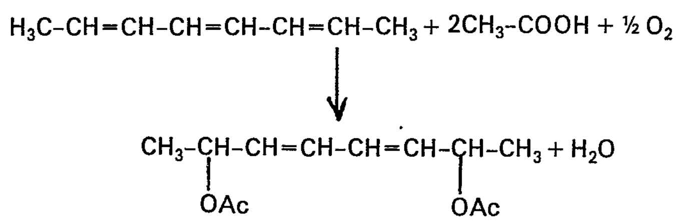

Es war nicht vorauszusehen, ob 1.3.5-Hexatriene überhaupt mit Sauerstoff und Carbonsäuren zu definierten Produkten reagieren und ob im Fall einer Reaktion überwiegend 1.6-Diacyloxy-2.4-hexadiene, 1.4-Diacyloxy-2.5-hexadiene oder 3.4-Diacyloxy-1.5-hexadiene gebildet würden. Auch war nicht vorauszusehen, ob die luftempfindlichen 1.3.5-Hexatriene unter den Reaktionsbedingungen in Gegenwart von Sauerstoff nicht weitgehend polymerisiert würden.

Nach dem Verfahren der Erfindung werden die Diacylate der Formel I aus den Hexatrienen der Formel II durch eine einstufige Acyloxilierung erhalten. Für die Herstellung von 1.6-Diacetoxy-2.4-hexadien benötigte man bisher eine mehrstufige Reaktion. So wird 1.5-Hexadien (Diallyl) mit N-Bromsuccinimid, 3.4-Dihydroxy-1.5-hexadien mit Phosphortribromid oder 1.3.5-Hexatrien mit Brom zu 1.6-Dibrom-2.4-hexadien bromiert, das bei der Umsetzung mit Silberacetat oder Natriumacetat in 1.6-Diacetoxy-2.4-hexadien übergeht [P. Karrer, W. Ringli, Helvetica Chimica Acta 30, 1771 (1947), Ch. Prévost, Comptes Rendus Hebdomadaires des Séances de l'Académie des Sciences 184, 458 (1927)].

Die nach der Erfindung erhältlichen Diacyloxyhexadiene der Formel

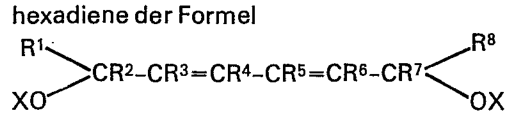 III,

in der X einen $R^9$-CO-Rest und die Reste $R^1$ bis $R^9$ Wasserstoffatome oder Alkylreste mit 1 bis 3 C-Atomen bedeuten, sind mit Ausnahme des 1.6-Diacetoxy-2.4-hexadiens neu.

Von besonderem technischen Interesse sind die neuen Diacyloxyhexadiene der Formel

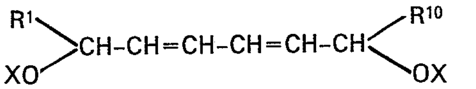

IV,

in der X einen $R^9$CO-Rest, $R^1$ und $R^9$ ein Wasserstoffatom oder einen Alkylrest mit 1 bis 3 C-Atomen und $R^{10}$ einen Alkylrest mit 1 bis 3 C-Atomen bedeuten, insbesondere die Verbindungen der Formel

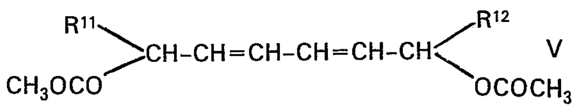

V

in der $R^{11}$ ein Wasserstoffatom, eine Methylgruppe oder eine Ethylgruppe und $R^{12}$ eine Methylgruppe oder eine Ethylgruppe bedeuten.

Als Ausgangsstoffe der Formel II seien beispielsweise 1.3.5-Hexatrien, 1.3.5-Heptatrien, 1.3.5-Octatrien oder 2.4.6-Octatrien genannt. Man kann die Triolefinen für sich oder als Mischungen, die z.B. auch noch andere Kohlenwasserstoffe, wie Monoolefine und Paraffinkohlenwasserstoffe enthalten, einsetzen.

Die genannten Ausgangsverbindungen lassen sich beispielsweise durch Abspaltung von Wasser aus entsprechenden Dienolen herstellen. So ist 1.3.5-Hexatrien durch Dehydratisierung von 1.3-Hexadien-1.5-ol, 1.3.5-Octatrien durch Dehydratisierung von 2.4-Octadien-6-ol zugänglich [K. Alder, H. v. Brachel, Liebigs Ann. Chem. 608, 195 (1957)]. 2.4.6-Octatrien ist beispielsweise durch Dimerisierung von Butatien zu 1.3.7-Octatrien (DE-OS 1443442) und anschliessende Isomerisierung mit Palladiumverbindungen [Jap. Patentanmeldung 50 601 (19727)] herstellbar.

Als Carbonsäuren kommen Ameisensäure, Essigsäure oder Propionsäure in Betracht.

Als Katalysatoren verwendet man Palladium, Platin oder Salze dieser Metalle, wobei diese Katalysatoren noch andere aktive Bestandteile enthalten können. Beispielsweise geeignet sind Trägerkatalysatoren, die als aktive Bestandteile, welche auf dem Trägermaterial aufgebracht sind, Palladium oder Platin und Kupfer und/oder Tellur enthalten.

Die für das erfindungsgemässe Verfahren geeigneten Katalysatoren lassen sich in bekannter Weise, z.B. nach den Angaben der DE-PS 2217452, DE-OS 2820519 oder DE-OS 2417558 herstellen. Katalysatoren der genannten Art enthalten beispielsweise, bezogen auf das Katalysatorgewicht, 1 bis 10% Palladium oder Platin, 0.1 bis 30% Kupfer und/oder 0.01 bis 10% Tellur. Bevorzugt ist die Verwendung eines Trägerkatalysators, der je Grammatom Palladium oder Platin 0.01 bis 6, vorzugsweise 1 bis 3.5 Grammatome Kupfer und/oder 0.01 bis 1, vorzugsweise 0.01 bis 0.4 Grammatome Tellur enthält.

Die Gesamtmenge der auf den Träger aufgebrachten katalytisch wirksamen Metalle beträgt, bezogen auf den Trägerkatalysator, zweckmässig z.B. 0.01 bis 30 Gew.-%. Grössere und kleinere Konzentrationen sind allerdings auch möglich.

Als Trägermaterial enthalten die Katalysatoren z.B. Aktivkohle, Bauxit, Bimsstein, Kieselgel, Kieselgur oder andere Formen der Kieselsäure, Magnesia, Ton oder Tonerde.

Man kann die katalytisch wirksamen Metalle z.B. auch ohne Trägermaterial anwenden, indem man sie in Form von Salzen im Reaktionsgemisch löst oder suspendiert.

Die Umsetzung zur Herstellung der Diacyloxyhexadiene nimmt man in an sich bekannter Weise in der Gas- oder Flüssigphase bei Temperaturen von 70 bis 180°C vor. In der Gasphase arbeitet man vorzugsweise bei 120 bis 150°C und in der Flüssigphase vorzugsweise bei 70 bis 110°C. Der Reaktionsdruck ist durch die Verfahrensweise gegeben und kann zwischen atmosphärischem Druck und z.B. 100 bar betragen. Das Verfahren kann chargenweise oder kontinuierlich z.B. mit fixiertem Bett, Wirbelbett oder Dreiphasenfliessbett durchgeführt werden.

Die nach dem Verfahren der Erfindung herstellbaren Diacyloxyhexadiene stellen wertvolle Ziwschenprodukte dar. Die aus ihnen nach Hydrierung und Verseifung hervorgehenden substituierten Hexandiole können wie das 1.6-Hexandiol (Ullmanns Encyklopädie, Band 7, Seite 228) und das 1.8-Octandiol (De-OS 1066566) beispielsweise für die Herstellung von Polyurethanen, Estern und Weichmachern verwendet werden.

Bei den Prozentangaben handelt es sich um Gew.-%.

Beispiel 1

a) Katalysatorherstellung

Man löst 89,6 g Kupferpulver in 660 $cm^3$ 33%iger Salpetersäure und vermischt diese Lösung bei Raumtemperatur mit der Lösung von 83,4 g $PdCl_2$ in 400 $cm^3$ eines warmen Gemisches aus 66%iger Salpetersäure und 32%iger Salzsäure (Volumenverhältnis 1:1) sowie mit der Lösung von 6,25 g $TeO_2$ in 1000 $cm^3$ warmer, 16%iger Salzsäure. Man legt 1000 g Aktivkohle (0,3 bis 0,5 mm) vor, die zuvor bei 70°C 5 Stunden mit 15%iger Salpetersäure gerührt, nach dem Abnutschen neutral gewaschen und bei 150°C unter Vakuum getrocknet worden war und fügt die vereinigte Metallsalzlösung hinzu. Anschliessend gibt man soviel Wasser hinzu, dass die Kohle völlig benetzt ist.

Dann wird am Rotationsverdampfer bei 85°C und unter Wasserstrahlvakuum zur Trockene eingedampft. Der Katalysator wird 2 Stunden bei 150°C im Vakuumtrockenschrank und dann 2 Stunden bei 150°C im Röhrenofen unter störmendem Stickstoff getrocknet. Anschliessend leitet man zur Aktivierung des Katalysators bei 400°C bei Raumtemperatur mit Methanol gesättigten Stickstoff und schliesslich 0,5 Stunden Wasser-

stoff (20 l/Stunde) bei 800°C über den Katalysator. Man lässt unter strömendem Stickstoff auf Raumtemperatur abkühlen.

Nach der Elementaranalyse enthält der Katalysator 5,04% Palladium, 8,2% Kupfer und 0,55% Tellur.

b) Acetoxilierung von Octatrien

Eine Apparatur, die aus einem 1-Liter-Dreihalskolben mit Anschützaufsatz, Tropftrichter, Begasungsrührer, Innenthermometer, Gaseinleitungsrohr und Rückflusskühler mit aufgesetztem Trokkeneiskühler besteht, wird mit Stickstoff gespült. Dann gibt man eine Suspension von 25 g des nach Beispiel 1a) hergestellten Katalysators in 543 g Eisessig in die Apparatur. Man erhitzt auf 95°C, leitet im Verlauf von 4 Stunden 12 l Sauerstoff ein und tropft gleichzeitig 54 g 2.4.6-Octatrien zu. Man leitet dann weitere 30 Minuten lang bei 95°C insgesamt 1,5 l Sauerstoff ein; anschliessend durchspült man die Apparatur 30 Minuten lang mit Stickstoff. Man lässt abkühlen und entfernt den Katalysator durch Filtrieren. Die anfallende Essigsäurelösung (603 g) wird am Rotationsverdampfer eingedampft. Durch fraktionierte Destillation des Rückstandes (92 g) erhält man 61,3 g eines Gemisches isomerer Diacetoxyoctadiene (54%, bezogen auf eingesetztes 2.4.6-Octatrien) vom Siedepunkt 104–113°C/1 mbar. Dieses Gemisch besteht nach gaschromatographischer Analyse zu 52% aus 2.7-Diacetoxy-3.5-octadien (1.6-Dimethyl-1.6-diacetoxy-2.4-hexadien), das beim Kühlen aus dem Gemisch auskristallisiert; Schmelzpunkt 74–75°C (aus Xylol).

Beispiel 2

In gleicher Weise wie in Beispiel 1b) beschrieben, werden in eine Suspension von 12,5 g eines nach der DE-OS 28 20 519 hergesellten Pd/Cu-Katalysators (5,27% Pd, 8,59% Cu) auf Aktivkohle als Träger in 543 g Essigsäure, innerhalb von 2 Stunden bei 95°C 6 l Sauerstoff eingeleitet und 26 g 1.3.5-Octatrien zugetropft. Man leitet dann weitere 30 Minuten lang bei 95°C insgesamt 1,5 l Sauerstoff ein; anschliessend durchspült man die Apparatur 30 Minuten lang mit Stickstoff. Nach der in Beispiel 1b) beschriebenen Aufarbeitung und fraktionierten Destillation der Essigsäurelösung (564 g) erhält man 23,9 g eines Gemisches isomerer Diacetoxyoctadiene (4%, bezogen auf eingesetztes 1.3.5-Octatrien) vom Siedepunkt 90–110°/0,5 mbar, in dem als Hauptprodukt 1.6-Diacetoxy-2.4-octadien (1.6-Diacetoxy-6-ethyl-2.4-hexadien) enthalten ist.

Beispiel 3

In gleicher Weise wie in Beispiel 1b) beschrieben, werden in eine Suspension von 12,5 g eines nach der DE-OS 22 17 452 hergestellten Pd/Te-Katalysators 65,27% Pd, 0,51% Te): auf Aktivkohle als Träger in 540 g Essigsäure innerhalb von 2 Stunden bei 95°C 6 l Sauerstoff eingeleitet und 18,6 g 1.3.5-Hexatrien zugetropft. Man leitet dann weitere 30 Minuten lang bei 95°C insgesamt 1,5 l Sauerstoff ein; anschliessend durchspült man die Apparatur 30 Minuten lang mit Stickstoff. Man erhält nach Aufarbeitung und Destillation 20,5 g eines Gemisches isomerer Diacetoxyhexadiene (44%, bezogen auf eingesetztes 1.3.5-Hexatrien) vom Siedepunkt 73–98°C/0,5 mbar. Nach gaschromatographischer Analyse besteht es zu 60% aus 1.6-Diacetoxy-2.4-hexadien, das beim Kühlen auskristallisiert; Schmelzpunkt 22–25°C (aus Diisopropylether).

## Patentansprüche

1. Verfahren zur Herstellung von Diacyloxyhexadienen der Formel

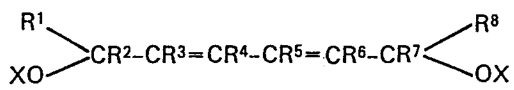

I,

in der X einen $R^9$-CO-Rest und die Reste R1 bis $R^9$ Wasserstoffatome oder Alkylreste mit 1 bis 3 C-Atomen bedeuten, dadurch gekennzeichnet, dass man Hexatriene der Formel

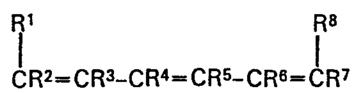

II

in Gegenwart von Katalysatoren, die Palladium, Platin oder Salze dieser Metalle enthalten, mit Sauerstoff und Carbonsäuren der Formel $R^9COOH$ umsetzt.

2. Verfahren nach Anspruch 1, dadurch gekennzeichnet, dass man die Umsetzung in der Gas- oder Flüssigphase bei Temperaturen von 70 bis 180°C vornimmt.

3. Diacyloxyhexadiene der Formel

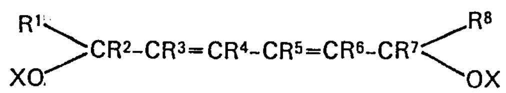

III,

in der X einen $R^9$-CO-Rest und die Reste $R^1$ bis $R^9$ Wasserstoffatome oder Alkylreste mit 1 bis 3 C-Atomen bedeuten, mit Ausnahme von 1.6-Diacetoxy-2.4-hexadien.

4. Diacyloxyhexadiene der Formel

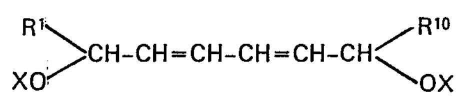

IV,

in der X einen $R^9CO$-Rest, $R^1$ und $R^9$ ein Wasserstoffatom oder einen Alyklrest mit 1 bis 3 C-Atomen und $R^{10}$ einen Alkylrest mit 1 bis 3 C-Atomen bedeuten.

5. Diacyloxyhexadiene der Formel

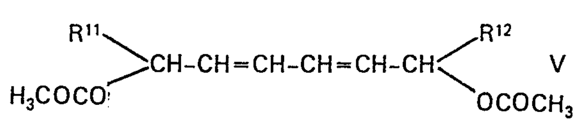

V

in der $R^{11}$ ein Wasserstoffatom, eine Methylgruppe oder eine Ethylgruppe und $R^{12}$ eine Methylgruppe oder eine Ethylgruppe bedeuten.

**Claims**

1. A process for the preparation of a diacyloxyhexadiene of the formula

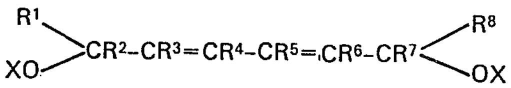 I,

where X is $R^9$-CO- and $R^1$ to $R^9$ are hydrogen or alkyl of 1 to 3 carbon atoms, wherein a hexatriene of the formula

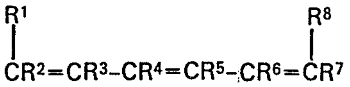 II

is reacted with oxygen and a carboxylic acid of the formula $R^9COOH$ in the presence of a catalyst which contains palladium or platinum or a salt of these metals.

2. A process as claimed in claim 1, wherein the reaction is carried out in the gas phase or liquid phase at from 70 to 180°C.

3. A diacyloxyhexadiene of the formula

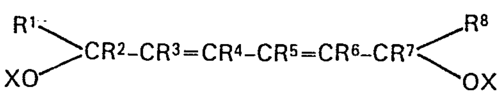 III,

where X is $R^9$-CO- and $R^1$ to $R^9$ are hydrogen or alkyl of 1 to 3 carbon atoms, with the exception of 1,6-diacetoxy-2,4-hexadiene.

4. A diacyloxyhexadiene of the formula

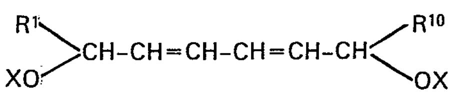 IV,

where X is $R^9$CO-, $R^1$ and $R^9$ are hydrogen or alkyl of 1 to 3 carbon atoms and $R^{10}$ is alkyl of 1 to 3 carbon atoms.

5. A diacyloxyhexadiene of the formula

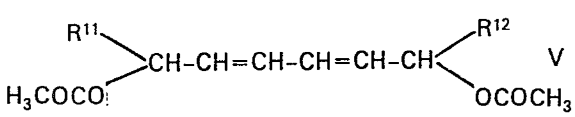 V

where $R^{11}$ is hydrogen, methyl or ethyl and $R^{12}$ is methyl or ethyl.

**Revendications**

1. Procédé pour la préparation de diacyloxyhexadiènes de formule

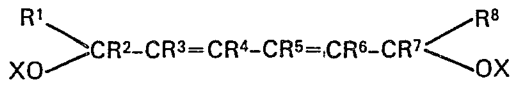 I,

dans laquelle X est un radical $R^9$-CO et les symboles $R^1$ à $R^9$ représentent des atomes d'hydrogène ou des radicaux alcoyle à 1-3 atomes de C, caractérisé en ce qu'on fait réagir des hexatriènes de formule

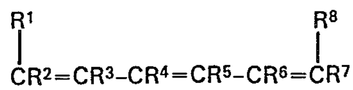 II

en présence de catalyseurs qui contiennent du palladium, du platine ou des sels de ces métaux, avec de l'oxygène et des acides carboxyliques de formule $R^9COOH$.

2. Procédé selon la revendication 1, caractérisé en ce qu'on effectue la réaction en phase gazeuse ou liquide à une température de 70 à 180°C.

3. Diacyloxyhexadiènes de formule

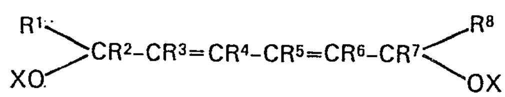 III,

dans laquelle X est un radical $R^9$-CO et les symboles $R^1$ à $R^9$ représentent des atomes d'hydrogène ou des radicaux alcoyle à 1-3 atomes de C, à l'exception du 1,6-diacétoxy-2,4-hexadiène.

4. Diacyloxyhexadiènes de formule

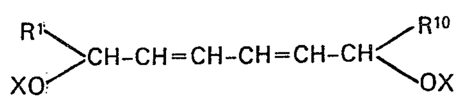 IV,

dans laquelle X est un radical $R^9$-CO, $R^1$ et $R^9$ représentent chacun un atome d'hydrogène ou un radical alcoyle à 1-3 atomes de C et $R^{10}$ un radical alcoyle à 1-3 atomes de C.

5. Diacyloxyhexadiènes de formule

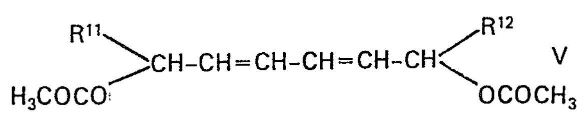 V

dans laquelle $R^{11}$ représente un atome d'hydrogène, un groupe méthyle ou un groupe éthyle et $R^{12}$ représente un groupe méthyle ou un groupe éthyle.